Europäisches Patentamt

European Patent Office

Office européen des brevets

(19)

(11) Publication number: **0 232 543**
A2

# (12) EUROPEAN PATENT APPLICATION

(21) Application number: **86118024.8**

(22) Date of filing: **23.12.86**

(51) Int. Cl.⁴: **A 61 F 2/06, A 61 L 27/00**

(30) Priority: **24.12.85 JP 296494/85**

(43) Date of publication of application: **19.08.87**
**Bulletin 87/34**

(84) Designated Contracting States: **DE FR GB**

(71) Applicant: **SUMITOMO ELECTRIC INDUSTRIES**
**LIMITED, No. 15, Kitahama 5-chome Higashi-ku,**
**Osaka-shi Osaka 541 (JP)**

(72) Inventor: **Okita, Koichi c/o Osaka Works, Sumimoto**
**Electric Ind. Ltd. 1-3, Shimaya 1-chome, Konohana-ku**
**Osaka-shi Osaka (JP)**
Inventor: **Asako, Shigeru c/o Osaka Works, Sumimoto**
**Electric Ind. Ltd. 1-3, Shimaya 1-chome, Konohana-ku**
**Osaka-shi Osaka (JP)**

(74) Representative: **Patentanwälte Grünecker, Kinkeldey,**
**Stockmair & Partner, Maximilianstrasse 58,**
**D-8000 München 22 (DE)**

(54) Tubular internal organ prosthetic material.

(57) A tubular internal organ prosthetic material comprising polytetrafluoroethylene tube (PTFE). The prosthetic material is characterized in that the fibrous constitution of the PTFE tube is oriented axially at an inner surface of said tube and is circumferentially oriented at an outer surface of said tube.

EP 0 232 543 A2

ACTORUM AG

TUBULAR INTERNAL ORGAN PROSTHETIC MATERIAL

BACKGROUND OF THE INVENTION

This invention relates to a tubular internal organ prosthetic material formed from a porous tube of polytetrafluoroethylene (PTFE) which material displays improved strength and integrity with surrounding tissue.

According to recent reports, porous PTFE tubes manufactured by the stretching method provide a suitable tubular internal organ prosthetic material, specifically for use as artificial blood vessels. Such a prosthetic material is said to be superior to conventional prosthetic materials which are knitted or woven. The stretched PTFE tube has a fine fibrous constitution having extremely fine fibrils and knots coupled together by the fine fibrils. The diameter of each fibril varies with the stretching conditions but may be significantly smaller than that of material which is knitted or woven. Moreover, the pore diameter and porosity of the tube may be varied. When the PTFE tube is used as an artificial blood vessel, it is soft and essentially free of thrombus. Further, the PTFE tube has excellent properties for forming the lumen of a blood vessel, and also does not injure adjoining tissue. PTFE tube is, therefore, an excellent tubular internal organ prosthetic material.

The stretched porous PTFE tube, however, tends to

- 1 -

tear when sutured to adjoining tissue. The porous PTFE tube often tears in the axial direction of the tube, due apparently to its fine fibrous consitutution which is strongly oriented in the axial direction. The tearing problem may be solved, by a process of spirally winding an additional porous PTFE tape or fiber, made of a different material, around the outer surface of the tube and integrating the two layers. The additional tape or fiber is intended to prevent the tube from tearing in the axial direction by providing the outer surface of the tube with an orientation perpendicular to the tube axis.

## SUMMARY OF THE INVENTION

It is an object of the present invention to provide a tubular internal organ prosthetic material having orientation in both the axial and the circumferential directions using a single tube. Thus, it is one object of the present invention to solve the tearing problem of conventional PTFE tube by causing the fibrous consititution to be continously variable from the inner surface, having a strong orientation in the axial direction, to the outer surface having a strong orientation in the circumferential direction.

A conventional porous PTFE tube will buckle and fail to keep a cylindrial profile if the tube is bent even with a small curvature. These characteristics are an obstacle to placing the tube into practical use. The tubular

- 2 -

0232543

internal organ prosthetic material according to the present invention, however, resists buckling due to the strong circumferential orientation of the outer surface. The conventional porous PTFE tube has a further problem in that, when it is used as an internal organ prosthetic material, integrity with surrounding tissue is inferior.

It is therefore another object of the present invention to provide a tubular internal organ prosthetic material characterized in that integrity with surrounding tissue is promoted by making the mean fibril length at the outer surface of the tube greater than that at the inner surface, thereby increasing the penetration and the integrity of the surrounding tissue into and with the prosthetic material.

### DETAILED DESCRIPTION OF THE INVENTION

The porous PTFE tube to which the present invention applies is prepared basically in accordnace with the method described in Japanese Patent Publication No. 13560/67. Fisrt, liquid lubricant is incorporated in unsintered PTFE powder, and the mixture is extruded by a ram extruder to a tubular form. After the liquid lubricant is removed from the tube or while it is being removed, the resulting tube is stretched in the axial direction. As the tube is stretched in the axial direction, its diameter may be expanded at the same time or in order. A tube offering

- 3 -

improved strength is obtained if the tube is sintered at a temperature of over 327°C without shrinking to fix the stretched structure. The porous PTFE tube thus obtained has a fine fibrous constitution with extremely thin fibrils and knots coupled together by the fibrils. Since the diameter and length of the fibril and the size and number of the knots can be vaired with the stretching and sintering conditions, the pore diameter and porosity of the porous tube obtained can readily be controlled. It has been clinically confirmed that when the tube is used as an internal organ prosthetic material e.g., as an artificial blood vessel, the mean fibril length should be preferably within the range of 10-100μm; the mean pore diameter between 1-100μm; the porosity over 70%; and the thickness of the tube 0.3-1.0 mm.

The porous PTFE tube thus obtained usually displays a fibrous constitution of PTFE having a strong orientation in the axial direction. The tube fixed in such a manner to prevent heat shrinkage is heated at over 327°C from its outer surface and sintered during the final sintering process until the outer surface is retieulated to provide a tubular internal organ prosthetic material according to the present invention. The mean fibril length at the outer surface is at least five times the mean fibril length at the inner surface of the prosthetic material. The mean knot thickness at the outer surface is at least ten times the mean knot thickness at the inner surface. The fibrous constitution within the wall of the PTFE tube varies continuously from the inner to the outer surface; the inner and outer surface of the prosthetic material being strongly oriented in the axial and circumferential directions, respectively.

To sinter the tube until the outer surface is reticulated, it should be heated at over 327°C from outside. In the fibrous PTFE constitution forming the tube, the fine fibrils at the outer surface are gradually cut and combined through fusion and the knots are also combined through fusion to make the fibril diameter greater so that longer fibrils and thicker knots are formed. By selecting the heat treatment temperature and the time, the relative fibril length and knot thickness to the inner surface can be controlled.

The present inventors have discovered that the penetration of and integrity with adjoining tissue are greatly improved when the implanted tubular internal organ prosthetic material has a mean fibril length at the outer surface at least five times the mean fibril length at the inner surface and when the mean knot thickness at the outer surface is at least ten times the mean knot thickness at the inner surface.

As a further preferable fibrous constitution, the mean fibril length at the inner surface should be in the range of 10μm - 100μm; at the outer surface, in the range of 50μm - 500μm; the mean knot thickness at the inner surface, in the range of 0.5μm - 5μm; and at the outer surface, in the range of 20μm - 200μm. Thus, the mean knot thickness at the outer surface should be at least 40 times that at the inner surface of the prosthetic material.

The most preferable fibrous constitution is characterized in that, the ratio of the thickness of the fibrous constitution at the outer surface to that of the fibrous constitution at the inner surface is in the range of from 1 : 1 to 9 : 1 thus making the fibrous constitution at the outer surface relatively thick. As the fibrous constitution at the outer surface becomes thick, the penetration of organic tissue becomes easier and the organization is also promoted.

The prosthetic material according to the present invention thus solves the tearing and buckling problems of conventional porous PTFE tube. Since the surrounding tissue readily penetrates the prosthetic material, integrity between the prosthetic material and surrounding tissue is improved.

As set forth above in detail, the tubular internal organ prosthetic material according to the present invention is extremely useful not only as an artificial blood vessel

- 6 -

but also as an artificial esophagus, windpipe, bilious pipe, ureter or the like.

The following Examples are provided for the purpose of describing the present invention in greater detail. It should, however, be understood that these Examples are for illustrative purposes only and are by no means to be considered as limiting.

## Example 1

Twenty-nine parts by weight of a liquid lubricant Deobase were added to 100 parts by weight of the fine PTFE powder Polyflont F-104 (of Daikin Kogyo) and uniformly mixed. The mixture was then extruded by a ram extruder into a tube 2 mm in inner diameter and 3.5 mm in outer diameter after it was press-preformed. The tube was dipped in trichloroethylene to extract the liquid lubricant and subsequently 400% stretched in the axial direction while it was being heated at about 280°C. The stretched tube was heated at about 380°C. By reducing pressure on the outer surface of the tube, the inner diameter was expanded to 3.0 mm thereby obtaining a porous PTFE tube. A stainless steel bar 3.0 mm in diameter was inserted in the tube, which the stretched tube was heated from the outer surface at 350°C for 30 minutes while both the ends of the tube were fixed to the bar. The tube was then cooled to the room temperature and the bar removed. A tube 3.0 mm in inner diameter, 3.8 mm in outer diameter, 70% in porosity, 40μm

- 7 -

in mean fibril length at the inner surface and 500µm in mean fibril length at the outer surface was thus obtained. A stainless steel wire 0.4 mm in diameter was passed from one end of the tube through the 5 mm tube wall to form a ring. When the tube was pulled at a speed of 50 mm/min in the axial direction, it was torn at a load of 3,800g, which was well over the 180g value obtained with a porous PTFE tube obtained by ordinary sintering.

### Example 2

The above-described mixture was extruded into a tube of 1.5 mm inner diameter, 2.5 mm outer diameter and was 200% stretched at 300°C in the axial direction. The pressure was reduced at the outer surface of the tube while heating at about 390°C to expand the inner diameter to 2.5 mm. The tube was fixed to prevent heat shrinkage and heated at 390°C for 20 minutes to obtain a tube with 69% porosity, 15µm in mean fibril length at the inner surface, 400µm in mean fiber length at the outer surface, 1.5µm in mean knot thickness at the inner surface and 200µm in mean knot thickness at the outer surface. The fibrous consitution at the outer surface accounted for 60% of the wall thickness.

### Example 3

A porous tube 2.0 mm in inner diameter was prepared by a method similar to that in Example 2. The tube was subjected to heat treatment at 390°C for 15 minutes. The tube thus obtained was 30μm in mean fibril length at the inner surface, 500μm in mean fibril length at the outer surface, 2μm in mean knot thickness at the inner surface and 180μm in mean knot thickness at the outer surface. The fibrous constitution at the outer surface accounted for 70% of the wall thickness.

WHAT IS CLAIMED IS:

1. A tubular internal organ prosthetic material comprising a polytetrafluoroethylene to be having a fibrous constitution with knots coupled together by fibrils, a mean fibril length at an outer surface of said material being at least five times a mean fibril length at an inner surface of said material; a mean knot thickness at said outer surface of said material being at least ten times a mean knot thickness at said inner suface; said fibrous constitution varying continuously from said inner surface to said outer surface; said inner and outer surfaces being strongly oriented axially and circumferentially, respectively.

2. A tubular internal organ prosthetic material as claimed in claim 1, wherein said fibrous constitution at said outer sarface is 50% to 90% of the thickness of a wall of said material.

3. A tubular internal organ prosthetic material as claimed in claim 1, wherein said mean fibril length at said inner surface is in the range of from 10 μm to 100 μm, and said mean fibril length at said outer surface is in the range of from 50 μm to 500 μm.

4. A tubular internal organ prosthetic material as claimed in claim 1, wherein said mean knot thickness at said inner surface is in the range of from 0.5 μm to 5 μm, and said mean knot thickness at said outer surface is in the range of from 20 μm to 200 μm.

5. A tubular internal organ prosthetic material as claimed in claim 1, wherein said mean knot thickness at said outer surface is about 40 times greater than said mean knot thickness at said inner surface.